# EUROPEAN PATENT SPECIFICATION

(11) **EP 2 135 598 B1**
(45) Date of publication and mention of the grant of the patent: **03.08.2011**
(21) Application number: 09156613.3
(22) Date of filing: 30.03.2009
(51) Int. Cl.: A61K 8/11, A61Q 17/04, C08F 2/44, C08F 2/24

(54) **Particle Containing Ultraviolet Absorber**
Partikel mit UV-Absorber
Particule contenant un absorbeur d'ultraviolets

(30) Priority: 16.06.2008 US 132180 P
(43) Date of publication of application: 23.12.2009
(73) Proprietor: Rohm and Haas Company, Philadelphia, PA 19106-2399 (US)
(72) Inventor: Jones, Charles Elwood, Yardley, PA 19067 (US); Wills, Morris Christopher, Philadelphia, PA 19152 (US)
(74) Representative: Kent, Venetia Katherine

(56) References cited:
- WO-A1-99/33439
- WO-A1-2005/023878
- WO-A1-2006/094526
- US-A- 5 945 090

## Description

This invention relates to a particle comprising a polymeric shell and an ultraviolet absorbing compound. Such particles are useful, e.g., in cosmetic and dermatological formulations.

Encapsulation of a variety of active ingredients in polymeric materials is known. For example, U.S. Pat. No. 7,176,255 teaches encapsulation of perylene visible dyes in a polymer shell by means of miniemulsion polymerization. However, methods for encapsulating ultraviolet absorbing compounds in small particles are not known.

The problem addressed by this invention is to provide a small, stable particle containing an ultraviolet absorbing compound.

### STATEMENT OF THE INVENTION

The present invention, in its various aspects, is as set out in the accompanying claims.

The present invention is directed to a particle having an average diameter from 80 to 500 nm and comprising: (a) a core comprising at least one compound having a λₘₐₓ in the range from 250 to 400 nm; and (b) a shell comprising a polymer comprising 50-100 wt % monomer residues of at least one multiethylenically unsaturated compound and 0-50 wt % monomer residues of at least one monoethylenically unsaturated compound, wherein the core is from 25 to 90 wt % of the particle, and the shell is from 10 to 75 wt % of the particle. The invention is further directed to a sunscreen formulation containing the particle and to a method for producing the particle by miniemulsion polymerization.

### DETAILED DESCRIPTION OF THE INVENTION

Percentages are weight percentages (wt %) and temperatures are in °C, unless specified otherwise. As used herein the term "(meth)acrylic" refers to acrylic or methacrylic. Monomers useful in this invention include monoethylenically unsaturated compounds and multiethylenically unsaturated monomers, i.e., crosslinkers, including, e.g., divinylaromatic compounds, di- and tri-(meth)acrylate esters, di- and tri-vinyl ether compounds, allyl (meth)acrylate. Monoethylenically unsaturated monomers include, e.g., (meth)acrylic acids and their esters, amides and nitriles; styrene, chloro- and/or methyl-substituted styrenes, vinylpyridines, and vinyl esters, ethers and ketones. Monomers which are sufficiently insoluble in an aqueous phase are preferred. The term "styrenic polymer" indicates a copolymer polymerized from monomers comprising a styrene monomer (substituted or unsubstituted styrene, e.g., styrene, α-methylstyrene, ethylstyrene) and/or at least one crosslinker, wherein the combined weight of styrene and crosslinkers is at least 50 weight percent of the total monomer weight. In some embodiments, a styrenic polymer is made from a mixture of monomers that is at least 75% styrene and crosslinkers, alternatively at least 90% styrene and divinylaromatic crosslinkers, alternatively from a mixture of monomers that consists essentially of styrene and at least one divinylaromatic crosslinker. In other embodiments, a styrenic polymer is made from a monomer mixture consisting essentially of at least one divinylaromatic crosslinker. The term "acrylic polymer" indicates a copolymer formed from a mixture of vinyl monomers containing at least one (meth)acrylic acid, ester or amide, or (meth)acrylonitrile, along with at least one crosslinker (e.g., allyl methacrylate, ALMA; trimethylolpropane trimethacrylate, TMPTMA), wherein the combined weight of the (meth)acrylic acid(s) (acrylic acid, AA; methacrylic acid, MAA) or ester(s) (e.g., methyl methacrylate, MMA) or amide(s) or (meth)acrylonitrile and the crosslinker(s) is at least 50 weight percent of the total monomer weight; preferably at least 75%, more preferably at least 90%, and most preferably from a mixture of monomers that consists essentially of at least one (meth)acrylic acid or ester and at least one crosslinker. In some embodiments, a (meth)acrylic acid ester is a C₂-C₄ hydroxy-substituted alkyl ester, e.g., 2-hydroxyethyl methacrylate (HEMA), 2-hydroxyethyl acrylate (HEA), 2-hydroxypropyl methacrylate (HPMA), 2-hydroxypropyl acrylate (HPA). An "acrylic/styrenic polymer" is one formed from a mixture of vinyl monomers comprising at least 50%, alternatively at least 75%, alternatively at least 90% of acrylic and styrenic monomers, as defined above.

The average particle size given for the particles of this invention is a weight average determined by light scattering measurements. In some embodiments of the invention, the average particle size is at least 100 nm, alternatively at least 125 nm, alternatively at least 150 nm, alternatively at least 160 nm. In some embodiments, the average particle size is no more than 400 nm, alternatively no more than 300 nm, alternatively no more than 250 nm, alternatively no more than 230 nm, alternatively no more than 220 nm, alternatively no more than 210 nm, alternatively no more than 200 nm.

At least one ultraviolet absorbing compound in the core preferably has λₘₐₓ of at least 275 nm, alternatively at least 300 nm. In some embodiments of the invention, at least two ultraviolet absorbing compounds are present in the core, one having λₘₐₓ in the range from 250 to 325 nm, and one having λₘₐₓ in the range from 325 to 400 nm. Preferably, ultraviolet absorbing compounds have an extinction coefficient at λₘₐₓ of at least 3,000 L/cm-mole, alternatively at least 4,000 L/cm-mol, alternatively at least 5,000 L/cm-mol. Preferably, ultraviolet absorbing compounds are organic compounds, i.e., those containing carbon and having at most trace levels of metals, more preferably aromatic compounds. Visible dyes having λₘₐₓ in the visible range, i.e., above 400 nm, are not considered ultraviolet absorbing compounds for the purposes of this invention. In some embodiments of the invention, the particle contains less than 1% of any visible dye, alternatively less than 0.5%, alternatively less than 0.2%, alternatively less than 0.1%, based on the weight of the particle. Examples of ultraviolet absorbing compounds may be found, e.g., in U.S. Pat. No. 7,316,809. Preferred ultraviolet absorbing compounds include, e.g., aminobenzoic acid, avobenzone, cinoxate, dioxybenzone, homosalate, menthyl anthranilate, octocrylene, octyl methoxycinnamate, octyl salicylate, oxybenzone, Padimate O, phenylbenzimidazole sulfonic acid, sulisobenzone and trolamine salicylate. A particularly preferred ultraviolet absorbing compound is avobenzone.

The shell polymer is a free-radical addition polymer. In some embodiments of the invention, it is an acrylic polymer, a styrenic polymer or an acrylic/styrenic polymer. In some embodiments of the invention, the shell polymer comprises at least 60% monomer residues of at least one multiethylenically unsaturated compound; and in some embodiments, no more than 95%, alternatively no more than 90%. In some embodiments of the invention, the shell polymer comprises no more than 40% monomer residues of at least one monoethylenically unsaturated compound; in some embodiments at least 5%, alternatively at least 10%. The aforementioned percentages are on the basis of the shell weight.

In some embodiments of the invention, preferably the monoethylenically unsaturated compound(s) used to make the shell polymer comprises at least one polar acrylic monomer, i.e., one having a hydroxy, carboxyl or amide group. Preferably the amounts of the polar acrylic monomer(s) are at least 1% based on polymer weight, alternatively at least 2%, alternatively at least 5%, alternatively at least 10%, alternatively at least 15%, alternatively at least 20%; preferably the amounts are no more than 50%, alternatively no more than 40%, alternatively no more than 30%, alternatively no more than 25%. Especially preferred monomers include HEMA, HPMA, HEA, HPMA, AA, MAA, acrylamide and methacrylamide. Preferably, the monoethylenically unsaturated compound(s) has a hydroxy group. Preferably, carboxylic acid monomer residues comprise no more than 5% of the polymer, alternatively no more than 3%, alternatively no more than 2%, alternatively no more than 1%. In some embodiments of the invention, the multiethylenically unsaturated compound(s) comprises at least one diethylenically unsaturated compound, e.g., allyl methacrylate (ALMA), allyl acrylate, divinylbenzene. In some embodiments of the invention, the multiethylenically unsaturated compound(s) comprises at least one triethylenically unsaturated compound, e.g., trimethylolpropane trimethacrylate.

In some embodiments of the invention, the core is at least 35% by weight of the particle, alternatively at least 45%, alternatively at least 50%, alternatively at least 55%, alternatively at least 60%, alternatively at least 65%, alternatively at least 70%, alternatively at least 75%, alternatively at least 80%; and in some embodiments no more than 85%. In some embodiments, the shell is at least 15% by weight of the particle; and in some embodiments, no more than 65%, alternatively no more than 55%, alternatively no more than 50%, alternatively no more than 45%, alternatively no more than 40%, alternatively no more than 35%, alternatively no more than 30%, alternatively no more than 25%, alternatively no more than 20%. In some embodiments of the invention, the core contains at least two ultraviolet absorbing compounds, one of which absorbs most strongly in the UV-A range (290-325 nm) and one of which absorbs most strongly in the UV-B range (325-400 nm). Preferably, at least one UV-A absorber is from 20-100% of the core, alternatively from 25-80%, alternatively from 25-60%, alternatively from 30-50%, alternatively from 30-40%, percentages on the basis of core weight. One preferred UV-A absorber is avobenzene, and one preferred UV-B absorber is homosalate (3,3,5-trimethylcyclohexyl salicylate). When avobenzone is present in the particle, preferably octyl methoxycinnamate is not present unless a uv stabilizer for avobenzone is also present. In some embodiments of the invention, the core contains a UV-A absorber and a solvent which does not necessarily absorb strongly in the ultraviolet range. The solvent preferably has a boiling point greater than 100°C at atmospheric pressure and is hydrophobic, i.e., it has an HLB (hydrophilic lipophilic balance) less than 10. One preferred solvent is isopropyl myristate.

The particle of this invention preferably is prepared by the technique of miniemulsion polymerization, as described, e.g., in G.H. Al-Ghamdi et al., J. Appl. Poly. Sci., vol. 101, pp. 3479-3486 (2006), or Landfester, K., Macromol. Rapid. Commun., vol. 22, pp. 896-936 (2001), and references cited therein, using an ultrasonic or high-pressure homogenizer, preferably with sufficient power to create 200 nm droplets. Preferably, polymerization is performed at temperatures ranging from 20°C to 100°C. Free-radical initiators are suitable for initiating the polymerization, including both water- and oil-soluble initiators, e.g., persulfate and lauroyl peroxide. Surfactants suitable for conventional emulsion polymerization are suitable for miniemulsion polymerization. Anionic surfactants are preferred. Preferably, the solids content of the polymerization mixture is from 20-65%, alternatively from 20-60%, alternatively from 25-55%, alternatively from 30-50%.

The present invention further comprises a cosmetic or dermatological formulation comprising the particle. Such formulations are well known, as described, e.g., in U.S. Pat. No. 6,379,683, and references cited therein. In some embodiments of the invention, the cosmetic or dermatological formulation contains particulate scatterers such as SUNSPHERES™ Polymer sold by the Rohm and Haas Company, metal oxides, solid UV absorbers, such as TINOSORB™ S and TINOSORB™ M sold by the Ciba Chemical Company, zinc oxide, titanium dioxide, or other scatterers such as glass beads or polymer particles. These would be included at a level to achieve maximum efficiency in scattering without providing whitening on the skin, unless desired for a skin muting effect in some formulations. The desirable level is generally a maximum of 5% and a minimum of 0.1% by weight in the formulation.

### EXAMPLES

### Example 1: General Miniemulsion Polymerization Procedure

The monomers, core compounds, initiator (persulfate), surfactant and water at a concentration of 30-50% solids are charged to a reactor. The mixture was agitated with an ultrasonic homogenizer at a starting temperature of 35°C, then heated to 75-85°C, and reached a maximum temperature due to exothermic reaction of about 95°C.

### Example 2: Preparation of Particles with Varying Cross-Linking

The procedure given above was used to prepare the following particles having varying cross-linking and shell content. Amounts of cross-linker (xl) and other monomers are relative to the shell polymer, and amounts of shell are relative to the entire particle. The core in particles B, C and E-G contained avobenzone and homosalate in a 31:69 weight ratio, and the core in particle H in a 33:67 weight ratio.

| | | Polymer Composition, % | | | | % Shell |
|---|---|---|---|---|---|---|
| ID | | TMPTMA | ALMA | MMA | MAA | |
| A | Control | | No polymer present | | | |
| B | 0% xl (Comparative) | 0.0 | 0.0 | 96.0 | 4.0 | 25% |
| C | 50% xl | 0.0 | 50.0 | 46.0 | 4.0 | 25% |
| D | Control | | No polymer present | | | |
| E | 2% xl (Comparative) | 0.0 | 2.0 | 96.0 | 2.0 | 50% |
| F | 20% xl (Comparative) | 0.0 | 20.0 | 78.0 | 2.0 | 50% |
| G | 50% xl | 50.0 | 0.0 | 48.0 | 2.0 | 50% |
| H | 100% xl | 0.0 | 100.0 | 0.0 | 0.0 | 25% |

### Example 3: Formulation and Testing

### OPTOMETRICS SPF 290 IN VITRO MEASUREMENT

Summary: The sunscreen to be tested is spread on a pre-cleaned 8.3 x 10.2 cm (3.25 x 4 in.) projector slide cover glass (KODAK cat # 140 2130) and the Sun Protection Factor (SPF) is estimated using SPF Operating Software (Version 2.1) and an SPF 290 Analyzer (The Optometrics Group).

### Procedure:

1. A 4" long piece is removed from a 3" roll of 3M TRANSPORE TapeR and placed on the sample holder supplied with the instrument, and the instrument is zeroed using this piece of tape on the holder.
2. Using a vacuum plate to hold the glass slide in place, a uniform film is drawn down on the glass slide using a either a 1.0 mil or a 1.5 mil spacer wet film applicator (Byk Gardner Company cat # SAR-5358) with a smooth motion at a moderate speed. The draw down speed must be consistent from determination to determination as the speed will effect the film thickness and hence the SPF value.
3. The sunscreen film is allowed to dry on the glass for at least 20 minutes. When multiple samples are being evaluated, the drawdowns of the samples are staged so the drying time for each sample is staggered and the measurement time after drawdown is consistent. While the samples are being dried, the OPTOMETRICS SPF 290 analyzer is started. After the instrument has been on for at least 20 minutes, the instrument is initialized with a blank piece of tape.
4. The blank piece of tape is placed on the sample holder and measured for UV absorbance as the background. (The software subtracts this absorbance from the sample absorbance to avoid measurement of the tape absorbance.)
5. After the films on the quartz glass have dried for 20 minutes, each glass plate with the sunscreen is placed on top of the tape (film side up) and then the holder with the sample is placed under the beam for measurement.
6. The sample is measured in six different areas on the glass slide. After recording all six readings, the OPTOMETRICS SPF 290 analyzer calculates an in vitro SPF value of the sunscreen emulsion. The final SPF is an average of these six determinations.

A sunscreen was formulated according to the following table. A typical laboratory overhead mixer (CAFRAMO BDC 2002) was used in the preparation. The procedure used was typical of sunscreen preparation with an aqueous phase being heated to 75°C, an oil phase being mixed to 75°C separately, mixing of the two phases and cooling with stirring to form the emulsion. Samples of miniencapsulated avobenzone were evaluated along with a 'control' sample containing an equal amount of avobenzone in the formulation added through a typical emulsion process used in preparing sunscreens.

| Component | A | B |
|---|---|---|
| Polymer→ | Control | sample formulation |
| Water, deionized | 84.50 | 66.23 |
| ACULYN 33 | 3.33 | 3.33 |
| Glycerin | 1.00 | 1.00 |
| Tetrasodium EDTA | 0.10 | 0.10 |
| particle G | 0.00 | 50.68 |
| Homosalate | 3.00 | 3.00 |
| Avobenzone | 1.50 | 0.00 |
| CERAPHYL 41 | 2.00 | 2.00 |
| GANEX V-220F | 1.50 | 1.50 |
| DOW CORNING 345 Fluid | 2.00 | 2.00 |
| Stearic Acid | 1.50 | 1.50 |
| Triethanolamine, 99% | 0.85 | 0.85 |
| | 100.00% | 100.00% |

The materials listed above with tradenames are materials typically used in personal care formulations and are listed in the INCI (International Nomenclature of Cosmetic Ingredients) Dictionary published by the Cosmetics, Toiletries and Fragrance Association, Washington, D.C. (11^{th} edition).

### Data for in vitro SPF measurements (data is with a 1.5 mil spacer)

All SPF measurements have been normalized to the control, which contains the same level of homosalate and avobenzone added through the typical sunscreen emulsification procedure (See procedure and formulations below).

| Sample | % SPF Boost | %UV Boost | Star Rating* |
|---|---|---|---|
| Control | 100 | 100 | 1 |
| Particle G | 315 | 257 | 4 |

| | | | |
|---|---|---|---|
| * The star rating is based on the average UVA/UVB ratio. This ratio is used as a measure of the relative absorbance of the sample in the UVA and UVB ranges. The UVA wavelength range is from 320 to 400 nm while the UVB range is from 290 nm to 3209 nm. This provides the area per unit wavelength of the absorbance curve from 320 nm to 400 nm divided by the area per unit wavelength of the absorbance curve from 290 nm to 320 nm. A different star rating is given to a range of values as shown below: | | | |

| UVA/UVB Ratio | Star Rating |
|---|---|
| <or=0.199 | 0 |
| 0.200 - 0.399 | 1 |
| 0.400 - 0.599 | 2 |
| 0.600-0.799 | 3 |
| >or=0.800 | 4 |

These two formulated sunscreen samples from above were submitted to IMS Inc. (Portland, ME) for in vitro photostability of the avobenzone, to compare the stability of the polymer encapsulated avobenzone with the avobenzone that had been added through normal emulsification. The samples were measured by an in vitro technique where the sunscreen is spread on VITRO-SKIN at 2 µl/cm², the standard application rate for sunscreens applied to the human body. The initial UVA/UVB ratio was measured and then the sample (on the VITRO-SKIN) was subjected to 5 MED (Minimal Erythermal Dose) from a 150 watt Xenon Arc Lamp (Solar Light Company). The UVA/UVB ratio was again determined. Then the sample was re-exposed to the 150 watt lamp for 10 more MED, for a total of 15 MED. The UVA/UVB ratio was again determined. The resultant data appear below.

| Sample exposure | Initial UVA/UVB Ratio | Ratio after 5 MED exposure | Ratio after 15 MED |
|---|---|---|---|
| Control sunscreen | 0.70 | 0.64 | 0.51 |
| Sunscreen with particle G | 0.83 | 0.83 | 0.75 |

The following sunscreens were formulated as described above.

| Component | Control A | particle B | particle C | Control B | particle E | particle F |
|---|---|---|---|---|---|---|
| Water, deionized | 84.50 | 66.23 | 66.23 | 82.89 | 55.47 | 55.47 |
| ACULYN 33 | 3.33 | 3.33 | 3.33 | 3.33 | 3.33 | 3.33 |
| Glycerin | 1.00 | 1.00 | 1.00 | 1.00 | 1.00 | 1.00 |
| Tetrasodium EDTA | 0.10 | 0.10 | 0.10 | 0.10 | 0.10 | 0.10 |
| particle B (30%) | 0.00 | 21.49 | 0.00 | 0.00 | 0.00 | 0.00 |
| particle C (30%) | 0.00 | 0.00 | 21.49 | 0.00 | 0.00 | 0.00 |
| particle E (30%) | 0.00 | 0.00 | 0.00 | 0.00 | 32.25 | 0.00 |
| particle F (30%) | 0.00 | 0.00 | 0.00 | 0.00 | 0.00 | 32.25 |
| Homosalate/ Avobenzone (69/31) | 3.22 | 0.00 | 0.00 | 4.83 | 0.00 | 0.00 |
| CERAPHYL 41 | 2.00 | 2.00 | 2.00 | 2.00 | 2.00 | 2.00 |
| GANEX V-220F | 1.50 | 1.50 | 1.50 | 1.50 | 1.50 | 1.50 |
| DOW CORNING 345 Fluid | 2.00 | 2.00 | 2.00 | 2.00 | 2.00 | 2.00 |
| Stearic Acid | 1.50 | 1.50 | 1.50 | 1.50 | 1.50 | 1.50 |
| Triethanolamine, 99% | 0.85 | 0.85 | 0.85 | 0.85 | 0.85 | 0.85 |
| | 100.00% | 100.00% | 100.00% | 100.00% | 100.00% | 100.00% |

| initial measurement | | | |
|---|---|---|---|
| Sample | % SPF boost | % UVA Boost | % xl in polymer |
| Control | 100 | 100 | |
| particle B (Comparative) | 378 | 300 | 0.0 |
| particle E (Comparative) | 153 | 137 | 2.0 |
| particle F (Comparative) | 196 | 167 | 20.0 |
| particle C | 366 | 239 | 50.0 |
| particle H | 407 | 285 | 100.0 |
| after storage at 45°C, one week | | | |
| Control | 100 | 100 | |
| particle B (Comparative) | 218 | 183 | 0.0 |
| particle E (Comparative) | 338 | 247 | 2.0 |
| particle F (Comparative) | 426 | 294 | 20.0 |
| particle C | 401 | 279 | 50.0 |
| particle H | 366 | 270 | 100.0 |

These measurements demonstrate that highly crosslinked particles provide better retention of uv-absorption properties after storage at elevated temperature.

### Example 4: Evaluation of the level of encapsulated avobenzone polymer

Sunscreens were formulated according to the following table. A typical laboratory overhead mixer (CAFRAMO BDC 2002) was used in the preparation. The procedure used was typical of sunscreen preparation with an aqueous phase being heated to 75°C, an oil phase being mixed to 75°C separately, mixing of the two phases and cooling with stirring to form the emulsion. One sample of miniencapsulated avobenzone was evaluated along with 'control' samples containing equal amounts of avobenzone in the formulation added through a typical emulsion process used in preparing sunscreens.

| Component | | | | | | |
|---|---|---|---|---|---|---|
| | 1.0% Avobenzone | 2.0% Avobenzone | 3.0% Avobenzone | part. H (1%) | part. H (2%) | part. H (3%) |
| water, deionized | 83.72 | 82.72 | 81.72 | 75.83 | 66.94 | 58.05 |
| Aculyn 33 | 3.33 | 3.33 | 3.33 | 3.33 | 3.33 | 3.33 |
| glycerin | 1.00 | 1.00 | 1.00 | 1.00 | 1.00 | 1.00 |
| tetrasodium EDTA | 0.10 | 0.10 | 0.10 | 0.10 | 0.10 | 0.10 |
| part. H (30%) | 0.00 | 0.00 | 0.00 | 8.89 | 17.78 | 26.67 |
| Homosalate | 3.00 | 3.00 | 3.00 | 3.00 | 3.00 | 3.00 |
| Parasol 1789 | 1.00 | 2.00 | 3.00 | 0.00 | 0.00 | 0.00 |
| Ceraphyl 41 | 2.00 | 2.00 | 2.00 | 2.00 | 2.00 | 2.00 |
| Ganex V-220F | 1.50 | 1.50 | 1.50 | 1.50 | 1.50 | 1.50 |
| Dow Corning 345 Fluid | 2.00 | 2.00 | 2.00 | 2.00 | 2.00 | 2.00 |
| stearic acid | 1.50 | 1.50 | 1.50 | 1.50 | 1.50 | 1.50 |
| triethanol-amine, 99% | 0.85 | 0.85 | 0.85 | 0.85 | 0.85 | 0.85 |
| | 100.00% | 100.00% | 100.00% | 100.00% | 100.00% | 100.00% |

Data for in vitro SPF measurements (data is with a 1.0 mil spacer) All SPF measurements have been normalized to the control which contains 1.0% avobenzone as stated above. All samples contain the same level of homosalate. The avobenzone in ID samples A through C is added through the typical sunscreen emulsification procedure. In ID samples D through F the avobenzone is added through the encapsulated polymer. The level of avobenzone in sample A is the same as sample D, sample B has the same level as sample E and sample C matches the level of avobenzone as sample F.

| ID | Sample | % SPF Boost | % UVA Boost | Star Rating |
|---|---|---|---|---|
| A | control (1% avobenzone) | 100 | 100 | 1 |
| B | 2% avobenzone | 161 | 164 | 1 |
| C | 3% avobenzone | 697 | 544 | 2 |
| D | part. H (8.89) | 407 | 285 | 1 |
| E | part. H (17.78) | 640 | 430 | 2 |
| F | part. H (26.67) | 1160 | 765 | 4 |

Since sample A contains the same amount of avobenzone as sample D, while sample B has the same amount as sample E and sample C has the same amount as sample F, comparisons between these sample pairings can be made to note the effect of encapsulation.

| ID | Sample | % SPF Boost Over appropriate Control | % UVA Boost Over appropriate Control |
|---|---|---|---|
| D | part. H (8.89) | 407 | 285 |
| E | part. H (17.78) | 396 | 260 |
| F | part. H (26.67) | 166 | 140 |

## Claims

1. A particle having an average diameter from 80 to 500 nm and comprising:
(a) a core comprising at least one compound having a λₘₐₓ in the range from 250 to 400 nm; and
(b) a shell comprising a polymer comprising 50-100 wt % monomer residues of at least one multiethylenically unsaturated compound and 0-50 wt % monomer residues of at least one monoethylenically unsaturated compound, wherein the core is from 25 to 90 wt % of the particle, and the shell is from 10 to 75 wt % of the particle.

2. The particle of claim 1 in which the core contains at least one ultraviolet absorbing compound having a λₘₐₓ in the range from 325 to 400 nm.

3. The particle of claim 2 in which the core contains at least two ultraviolet absorbing compounds, one of which absorbs most strongly in the range from 290 to 325 nm and one of which absorbs most strongly in the range from 325 to 400 nm.

4. The particle of claim 3 in which the particle has an average diameter from 125 to 250 nm.

5. A sunscreen formulation comprising the particle of claim 1.

6. A method for producing an ultraviolet absorbing particle; said method comprising steps of:
(a) providing at least one compound having a λₘₐₓ in the range from 250 to 400 nm;
(b) providing 0-50 wt % of at least one monoethylenically unsaturated monomer and 50-100 wt % of at least one multiethylenically unsaturated monomer, wherein percentages are based on total monomer;
(c) forming in an aqueous medium a miniemulsion having an average particle size from 80 to 500 nm and a solids content from 20-65 wt %; and
(d) polymerizing said at least one monoethylenically unsaturated monomer and at least one multiethylenically unsaturated monomer,
wherein said at least one compound is from 25 to 90 wt % of the particle, and said at least one monoethylenically unsaturated monomer and at least one multiethylenically unsaturated monomer are from 10 to 75 wt % of the particle.

## Patentansprüche

1. Teilchen mit einem mittleren Durchmesser von 80 bis 500 nm und umfassend:
(a) einen Kern, umfassend mindestens eine Verbindung mit einem λₘₐₓ im Bereich von 250 bis 400 nm, und
(b) eine Schale, umfassend ein Polymer, umfassend 50 bis 100 Gew.-% Monomerreste von mindestens einer mehrfachethylenisch ungesättigten Verbindung und 0 - 50 Gew.-% Monomerreste von mindestens einer monoethylenisch ungesättigten Verbindung,
wobei der Kern 25 bis 90 Gew.-% des Teilchens ausmacht und die Schale 10 bis 75 Gew.-% des Teilchens ausmacht.

2. Teilchen nach Anspruch 1, in welchem der Kern mindestens eine ultraviolettabsorbierende Verbindung mit einem λₘₐₓ im Bereich von 325 bis 400 nm enthält.

3. Teilchen nach Anspruch 2, in welchem der Kern mindestens zwei ultraviolettabsorbierende Verbindungen enthält, von denen eine am stärksten im Bereich von 290 bis 325 nm absorbiert und von denen eine am stärksten im Bereich von 325 bis 400 nm absorbiert.

4. Teilchen nach Anspruch 3, in welchem das Teilchen einen mittleren Durchmesser von 125 bis 250 nm aufweist.

5. Sonnenschutzformulierung, umfassend das Teilchen nach Anspruch 1.

6. Verfahren zum Herstellen eines ultraviolett-absorbierenden Teilchens, wobei das Verfahren die Schritte umfaßt:
(a) Bereitstellen mindestens einer Verbindung mit einem λₘₐₓ im Bereich von 250 bis 400 nm,
(b) Bereitstellen von 0 bis 50 Gew.-% mindestens eines monoethylenisch ungesättigten Monomers und 50 bis 100 Gew.-% mindestens eines mehrfachethylenisch ungesättigten Monomers, wobei die prozentualen Angaben auf der Gesamtmenge an Monomer basieren,
(c) Bilden einer Miniemulsion mit einer mittleren Teilchengröße von 80 bis 500 nm und einem Feststoffgehalt von 20-65 Gew.-% in einem wässrigen Medium, und
(d) Polymerisieren des mindestens einen monoethylenisch ungesättigten Monomers und mindestens einen mehrfachethylenisch ungesättigten Monomers, wobei die mindestens eine Verbindung 25 bis 90 Gew.-% des Teilchens beträgt, und das mindestens eine monoethylenisch ungesättigte Monomer und das mindestens eine mehrfachethylenisch ungesättigte Monomer 10 bis 75 Gew.-% des Teilchens betragen.

## Revendications

1. Particule ayant un diamètre moyen de 80 à 500 nm et comprenant :
(a) un noyau comprenant au moins un composé ayant une λₘₐₓ dans l'intervalle de 250 à 450 nm ; et
(b) une enveloppe comprenant un polymère comprenant 50-100 % en poids de résidus monomères d'au moins un composé multiéthyléniquement insaturé et 0-50 % de résidus de monomères d'au moins un composé monoéthyléniquement insaturé,
dans laquelle le noyau constitue de 25 à 90 % en poids de la particule, et l'enveloppe constitue de 10 à 75 % en poids de la particule.

2. Particule selon la revendication 1, dans laquelle le noyau contient au moins un composé absorbant les ultraviolets ayant une λₘₐₓ dans l'intervalle de 325 à 400 nm.

3. Particule selon la revendication 2, dans laquelle le noyau contient au moins deux composés absorbant des ultraviolets, dont l'un absorbe le plus fortement dans l'intervalle de 290 à 325 nm et dont l'un absorbe le plus fortement dans l'intervalle de 325 à 400 nm.

4. Particule selon la revendication 3, dans laquelle la particule présente un diamètre moyen de 125 à 250 nm.

5. Formulation d'écran solaire comprenant la particule selon la revendication 1.

6. Procédé de production d'une particule absorbant les ultraviolets ; ledit procédé comprenant les étapes consistant :
(a) à fournir au moins un composé présentant une λₘₐₓ dans l'intervalle de 250 à 400 nm ;
(b) à fournir 0-50 % en poids d'au moins un monomère monoéthyléniquement insaturé et 50-100 % en poids d'au moins un monomère multiéthyléniquement insaturé, dans lequel les pourcentages sont rapportés au monomère total ;
(c) à former dans un milieu aqueux une mini-émulsion ayant une taille moyenne de particules de 80 à 500 nm et une teneur en matières solides de 20-65 % en poids ; et
(d) à polymériser lesdits au moins un monomère monoéthyléniquement insaturé et au moins un monomère multiéthyléniquement insaturé,
dans lequel ledit au moins un composé constitue de 25 à 90 % en poids de la particule, et lesdits au moins un monomère monoéthyléniquement insaturé et au moins un monomère multiéthyléniquement insaturé constituent de 10 à 75 % en poids de la particule.
